Europäisches Patentamt

European Patent Office

Office européen des brevets

(19)

(11) Veröffentlichungsnummer : **0 460 127 B1**

## (12) EUROPÄISCHE PATENTSCHRIFT

(45) Veröffentlichungstag der Patentschrift :
**20.01.93 Patentblatt 93/03**

(51) Int. Cl.$^5$ : **A61K 7/13**

(21) Anmeldenummer : **90916193.7**

(22) Anmeldetag : **26.10.90**

(86) Internationale Anmeldenummer :
**PCT/EP90/01904**

(87) Internationale Veröffentlichungsnummer :
**WO 91/09588 11.07.91 Gazette 91/15**

(54) **SCHAUMFÖRMIGES HAARTÖNUNGSMITTEL.**

(30) Priorität : **21.12.89 DE 3942315**

(43) Veröffentlichungstag der Anmeldung :
**11.12.91 Patentblatt 91/50**

(45) Bekanntmachung des Hinweises auf die
Patenterteilung :
**20.01.93 Patentblatt 93/03**

(84) Benannte Vertragsstaaten :
**DE ES FR GB IT**

(56) Entgegenhaltungen :
**EP-A- 0 150 765**
**EP-A- 0 182 330**
**EP-A- 0 184 061**

(73) Patentinhaber : **Wella Aktiengesellschaft**
**Berliner Allee 65**
**W-6100 Darmstadt (DE)**

(72) Erfinder : **CLAUSEN, Thomas**
**Ernst-Pasqué-Stra e 35 A**
**W-6146 Alsbach (DE)**
Erfinder : **HOFFMANN, Axel**
**An der Lettkaute 16**
**W-6107 Reinheim 1 (DE)**

## Beschreibung

Gegenstand der Erfindung ist ein schaumförmiges Haartönungsmittel, insbesondere für den Naturtonbereich, welches ein verbessertes Deckvermögen aufweist und eine größere Farbtiefe ermöglicht.

Zum Färben von Haaren werden üblicherweise Oxidationshaarfärbemittel verwendet. Hierbei werden zur Entwicklung der Farbstoffe Oxidationsmittel, wie zum Beispiel Wasserstoffperoxid, benötigt. Durch die Einwirkung des Wasserstoffperoxids auf das Haar wird dieses gebleicht, was für die Erzielung bestimmter Farbnuancen unerwünscht ist. Gleichzeitig wird das Haar in seiner Struktur geschädigt.

Neben Oxidationshaarfärbemitteln werden Haartönungsmittel verwendet, welche direktfärbende Farbstoffe als Grundlage haben. Die Haartönungsmittel weisen den Vorteil auf, die Haarstruktur weniger zu belasten, da sie ohne Oxidationsmittel und ohne größere Mengen an Alkali auskommen. Jedoch haben die Haartönungsmittel deutliche Nachteile, da die Deckkraft sowie das Ausgleichsvermögen, insbesondere bei grauem oder teilweise ergrautem Haar, nicht ausreichend ist. Dadurch ist bei Haartönungsmitteln auch die Färbung der Haare in dunklen Naturtönen stark eingeschränkt.

Die Haartönungsmittel werden üblicherweise in Form einer Lösung oder einer Creme auf das Haar aufgetragen. In letzter Zeit wurden auch schaumförmige Haartönungsmittel vorgeschlagen.

Derartige schaumförmige Haartönungsmittel weisen jedoch infolge der schlechteren Benetzung der Haaroberfläche schlechtere Färbeeigenschaften auf.

Es bestand daher die Aufgabe, ein schaumförmiges Haartönungsmittel zur Verfügung zu stellen, welches eine intensivere Deckkraft und ein besseres Ausgleichsvermögen besitzt als bekannte schaumförmige Haartönungsmittel und welches auch die Erzielung von dunklen Naturtönen ermöglicht.

Als Lösung dieser Aufgabe, wurde nun gefunden, daß ein Mittel zum Tönen von Haaren, auf der Basis einer wäßrigen oder wäßrig-alkoholischen Farbstoffträgermasse und darin gelösten direktfärbenden Haarfarbstoffen, welches in Schaumform angewendet wird und welches

0,5 - 5,0 Gewichtsprozent eines $C_{14}$-$C_{18}$-Fettalkohols oder eines Gemisches aus $C_{14}$-$C_{18}$-Fettalkoholen und

0,1 - 1,5 Gewichtsprozent eines $C_8$-$C_{16}$-Alkylsulfats oder eines mit 1 bis 4 Ethylenoxideinheiten oxethylierten $C_8$-$C_{16}$-Alkylethersulfats
sowie

0,1 - 1,5 Gewichtsprozent 4-N-Ethyl-1,4-bis-[(2'-hydroxyethyl)-amino]-2-nitrobenzol,

0,01 - 0,25 Gewichtsprozent 4-(2'-Ureidoethylamino)-nitrobenzol und

0,01 - 0,5 Gewichtsprozent 4-(2'-Hydroxyethylamino)-3-nitrotoluol
enthält, wobei die Gewichtsprozentangaben sich jeweils auf die Gesamtmenge des Mittels beziehen, die gestellte Aufgabe in hervorragender Weise löst.

Alle Gewichtsprozentangaben dieser Anmeldung beziehen sich auf das Haartönungsmittel ohne Treibmittel.

Das erfindungsgemäße Haartönungsmittel ermöglicht es, graue Haare mit einem Weißanteil von 35 Prozent, in bestimmten Fällen - abhängig von der Haarstruktur - bis zu einem Weißanteil von 50 Prozent, gleichmäßig und mit guter Deckkraft zu färben. Bei Einwirkung von Wärme während des Färbevorgangs werden sogar gleichmäßige Färbungen bis zu einem Weißanteil von 80 Prozent erzielt.

Die hervorragenden Färbeeigenschaften des erfindungsgemäßen Haartönungsmittels sind völlig überraschend. Im Vergleich zu einem entsprechenden üblichen Haartönungsmittel in Cremeform wird mit dem neuen Haartönungsmittel die gleiche oder in bestimmten Fällen sogar eine bessere Farbtiefe erzielt. Dieses Ergebnis war nicht zu erwarten, da bei einem schaumförmigen Haartönungsmittel die Haaroberfläche schlechter benetzt wird, wodurch sich Nachteile in bezug auf Farbtiefe und Deckkraft ergeben.

Daß die große Farbtiefe und Deckkraft des erfindungsgemäßen Haartönungsmittels überraschend sind, läßt sich durch einen Vergleich mit einem schaumförmigen Haartönungsmittel herkömmlicher Zusammensetzung demonstrieren (vergleiche Beispiel 1).

So werden mit der Farbstoffkombination des neuen Haartönungsmittels beim Einsatz in gleicher Menge in einem üblichen Schaumtönungsmittel nur sehr viel weniger intensive Haarfärbungen erzielt. Die erfindungsgemäße Kombination aus einer geeigneten Trägermasse mit besonders ausgewählten Farbstoffen ermöglicht damit erstmals, die guten Färbeeigenschaften eines cremeförmigen Haartönungsmittels auch mit einem schaumförmigen Haartönungsmittel zu erreichen oder sogar zu übertreffen.

Im Vergleich zu cremeförmigen Haartönungsmitteln gleicher Farbtiefe ergeben sich für das erfindungsgemäße schaumförmige Haartönungsmittel eine Reihe von Vorteilen für den Anwender:

- Der kompakte Schaum läßt sich gut ohne Hilfsmittel in die Haare einarbeiten.
- Das Abtropfen flüssiger Färbemasse oder das Abrutschen von Creme von den Haaren wird vermieden. Dadurch wird auch die Anschmutzung von Kleidungsstücken vermieden.

2

- Bei der Anwendung in Schaumform besitzt das aufgetragene Haartönungsmittel ein größeres Volumen, wodurch die Verteilung und die Einarbeitung der Färbemasse erleichtert wird.

- Das schaumförmige Mittel ist im Vergleich zu einer Creme nach der Anwendung besser ausspülbar.

- Durch den geringeren Anteil an Trägerstoffen wird im Vergleich zu einer Creme die Belastung der Haare verringert.

Hinsichtlich der färberischen Möglichkeiten bietet das erfindungsgemäße Haartönungsmittel je nach Art und Zusammensetzung der Farbkomponenten eine färberische Abdeckung des gesamten Naturtonbereichs, der von natürlichen Farbtönen bis hin zu modischen Nuancen reichen kann.

Das neue Haartönungsmittel kann zusätzlich 0,1 - 15 Gewichtsprozent eines niederen aliphatischen Alkohols, vorzugsweise Ethanol oder Isopropanol enthalten. Der in dem erfindungsgemäßen Haartönungsmittel enthaltene Fettalkohol oder das Gemisch aus Fettalkoholen ist vorzugsweise ausgewählt aus $C_{16}$-$C_{18}$-Fettalkoholen und ist vorzugsweise in einer Menge von 0,5 - 3,0 Gewichtsprozent enthalten.

Als Alkylsulfat oder Alkylethersulfat kommt vorzugsweise ein $C_{10}$-$C_{14}$-Alkylsulfat oder $C_{10}$-$C_{14}$-Alkylethersulfat in Betracht, welches vorzugsweise in einer Menge von 0,1 bis 1,0 Gewichtsprozent enthalten ist.

Selbstverständlich kann das erfindungsgemäße Haartönungsmittel weitere direktfärbende Farbstoffe enthalten, beispielsweise 4-[Bis-(2′-hydroxyethyl)-amino]-1-(2′-hydroxyethylamino)-2-nitrobenzol, 4-(2′,3′-Dihydroxypropylamino)-3-nitro-trifluormethylbenzol, 4-(2′,3′-Dihydroxypropylamino)-5-chlor-2-nitroanilin, 4-(3′-Hydroxypropylamino)-3-nitrophenol, 1-[4′-Bis-[(2″-hydroxyethyl)-amino]-phenylazo]-4-aminobenzol, 2-Amino-4,6-dinitrophenol, 1-Amino-2-(2′-hydroxyethyl)amino-5-nitrobenzol, 1,4,5,8-Tetraaminoanthrachinon, 1-(2′-Hydroxy-4′-sulfo-6′-nitro)naphthylazo-2-hydroxy-naphthalin, 1,4-Diaminoanthrachinon, 1,4-Diamino-2-methoxyanthrachinon sowie 1-Methylamino-4-(2′-hydroxyethylamino)anthrachinon.

Weitere für solche Haartönungsmittel übliche Zusätze sind quaternäre Polymere, wie zum Beispiel Copolymerisate aus Vinylpyrrolidon und Dimethylaminoethylmethacrylat oder ein quaternisiertes Polymerisat aus Dimethylaminomethylmethacrylat, welche die Kämmbarkeit und den Glanz der Haare verbessern sowie einen konditionierenden Effekt ermöglichen.

Darüberhinaus können in dem neuen Mittel auch Lösungsmittel wie Wasser, Glycerin oder Glykole, wie zum Beispiel Ethylenglykol und Propylenglykol, oder auch Glykolether, weiterhin Netzmittel oder Emulgatoren aus den Klassen der anionischen, kationischen, amphoteren oder nichtionogenen oberflächenaktiven Substanzen, wie zum Beispiel Alkylsulfonate, Alkyltrimethylammoniumsalze, Alkylbetaine, oxehylierte Fettalkohole, Fettsäurealkanolamide, oxethylierte Fettsäureester, ferner Verdicker, Stärke, Polyacrylsäure, Cellulosederivate wie zum Beispiel Carboxymethylcellulose, Alginate, Vaseline, Paraffinöle und Fettsäuren sowie außerdem Pflegestoffe, wie zum Beispiel Lanolinderivate, Cholesterin, Pantothensäure und Betain, weiterhin Parfümöle und Komplexbildner enthalten sein. Die erwähnten Bestandteile werden in den für solche Zwecke üblichen Mengen verwendet, zum Beispiel die Netzmittel und Emulgatoren in Konzentrationen von etwa 0,05 bis 3,0 Gewichtsprozent, während die Verdicker in einer Menge von etwa 0,01 bis 0,5 Gewichtsprozent in den Zubereitungen enthalten sein können.

Das erfindungsgemäße Haartönungsmittel weist vorzugsweise einen pH-Wert von 4 bis 10 auf und wird in einen Behälter abgefüllt aus dem es durch eine geeignete Vorrichtung in Form eines Schaumes entnommen werden kann. Vorzugsweise liegt das erfindungsgemäße Haartönungsmittel als Aerosolpräparat vor, wobei es zusammen mit einem geeigneten Treibgas im Verhältnis 20:1 bis 1:1 in einem Druckbehälter abgefüllt ist und aus diesem in Form eines Aerosolschaumes entnommen wird.

Als geeignete Treibgase kommen beispielsweise halogenierte Kohlenwasserstoffe, leichtflüchtige aliphatische Kohlenwasserstoffe, wie zum Beispiel Propan oder Butan und Dimethylether in Betracht, wobei Propan, Butan und Dimethylether oder Gemische dieser Verbindungen aufgrund der Umweltschädlichkeit der halogenierten Kohlenwasserstoffe bevorzugt sind.

Die Anwendung des erfindungsgemäßen Haartönungsmittels erfolgt in bekannter Weise, indem man eine zur Tönung des Haares ausreichende Menge des vorstehend genannten Mittels, im allgemeinen etwa 15 bis 70 g, auf das Haar aufträgt, sodann den Schaum in das Haar einarbeitet, und nach einer Einwirkungsdauer von 5 bis 30 Minuten bei einer Temperatur von 15 bis 50 Grad Celsius das Haar mit Wasser spült und trocknet.

Die nachfolgenden Beispiele sollen den Gegenstand der Erfindung näher erläutern, ohne ihn auf diese Beispiele zu beschränken.

**Beispiele**

**Beispiel 1: Vergleichsversuch zur Bestimmung der Farbtiefe bei Verwendung gleicher Farbstoffe in verschiedenen Zusammensetzungen**

**Beispiel 1a: Haartönungsmittel in Cremeform**

```
6,30 g Cetylstearylalkohol
0,10 g p-Hydroxybenzoesäuremethylester
1,90 g Laurylsulfat-Natriumsalz
0,30 g 4-N-Ethyl-1,4-bis-[(2'-hydroxyethyl)-amino]-2-
       nitrobenzol
0,05 g 4-(2'-Ureidoethylamino)-nitrobenzol
0,06 g 4-(2'-Hydroxyethylamino)-3-nitrotoluol
91,29 g Wasser
100,00 g
```

94 g des vorstehenden Haartönungsmittels werden vor Gebrauch mit 6 Gewichtsprozent Wasser vermischt, um die gleiche Endkonzentration an Farbstoffen wie bei den Aerosols-Produkten 1b und 1c einzustellen. Das Mittel wird auf weiße Haare aufgetragen. Man läßt es 20 Minuten lang bei 37 Grad Celsius einwirken und spült dann das Haar mit Wasser. Anschließend wird das Haar getrocknet. Es ist in einem rötlich-aschigen natürlichen Farbton gefärbt.

**Beispiel 1b: Haartönungsmittel in Schaumform (herkömmliche Formulierung)**

```
4,50 g  Laurylalkoholdiglykolethersulfat-Natriumsalz,
        28prozentige wäßrige Lösung
3,00 g  Laurylalkoholdiglykolether
0,30 g  4-N-Ethyl-1,4-bis-[(2'-hydroxyethyl)-amino]-2-
        nitrobenzol
0,05 g  4-(2'-Ureidoethylamino)-nitrobenzol
0,06 g  4-(2'-Hydroxyethylamino)-3-nitrotoluol

92,09 g  Wasser

100,00 g
```

Die vorstehende Wirkstoff-Lösung wird im Verhältnis 94 : 6 zusammen mit Propan/Butan (2,7 bar) in eine Aerosoldose abgefüllt und als Schaum entnommen. Weiße Haare werden wie in Beispiel 1a mit dem Mittel behandelt und zeigen nach dem Trocknen einen im Vergleich zu Beispiel 1a unnatürlichen violett-aschigen Farbton mit deutlich geringerer Farbtiefe.

**Beispiel 1c: Haartönungsmittel in Schaumform (erfindungsgemäßes Haartönungsmittel)**

| | |
|---|---|
| 1,20 g | Cetylstearylalkohol |
| 0,10 g | p-Hydroxybenzoesäuremethylester |
| 0,35 g | Laurylsulfat-Natriumsalz |
| 0,30 g | 4-N-Ethyl-1,4-bis-[(2'-hydroxyethyl)-amino]-2-nitrobenzol |
| 0,05 g | 4-(2'-Ureidoethylamino)-nitrobenzol |
| 0,06 g | 4-(2'-Hydroxyethylamino)-3-nitrotoluol |
| 97,94 g | Wasser |

100,00 g

Die vorstehende Wirkstoff-Lösung wird im Verhältnis 94 : 6 zusammen mit Propan/Butan (2,7 bar) in eine Aerosoldose abgefüllt. Weiße Haare werden wie in Beispiel 1a mit dem Mittel behandelt und zeigen nach dem Trocknen im Vergleich zu Beispiel 1a einen vergleichbaren Farbton bei etwa gleicher Farbtiefe.

Farbmeßwerte:

Die Farbmeßwerte der nach den Beispielen 1a - 1c gewonnenen Haarfärbungen wurden mit einem Minolta Chromameter II ermittelt.

Man erhält folgende Meßwerte:

| Beispiel | Y | x | y | z(berechnet) |
|---|---|---|---|---|
| 1 a | 6,4 | 0,363 | 0,338 | 0,299 |
| 1 b | 9,6 | 0,341 | 0,314 | 0,345 |
| 1 c | 6,0 | 0,361 | 0,333 | 0,306 |

Bei der Angabe der Farbmeßwerte im oben benutzten Yxyz-System steht der Wert Y für die Farbtiefe der Färbung. Da Y den Prozentanteil des reflektierten Lichts angibt, entsprechen hohe Werte sehr hellen Proben und kleinere Werte dunklen bis schwarzen Proben.

Der x-Wert ist ein Relativwert für den Rotanteil der Probe (0 - 1,0), wobei kleinere Werte einem kleineren Rotanteil und große Werte einem großen Rotanteil entsprechen. Für die y- und z-Werte gilt entsprechendes. y ist ein Maß für den Grünanteil der Probe, während z den Blauanteil angibt.

Prinzipiell gilt die Formel $x + y + z = 1$, so daß sich die Einzelwerte normalerweise zwischen 0.2 und 0.5 bewegen. Während die x- und y-Werte gemessen werden läßt sich der z-Wert durch die Formel $z = 1-(x + y)$ berechnen.

Die Meßwerte zeigen die auch visuell beobachtete Tendenz:

Die Färbung mit dem erfindungsgemäßen schaumförmigen Haartönungsmittel 1c weist eine etwas größere Farbtiefe als die Färbung mit dem cremeförmigen Haartönungsmittel 1a auf, während die Farbtiefe mit dem herkömmlichen Schaumtönungsmittel 1b deutlich geringer ist.

Die gleiche Tendenz wird bei den Meßwerten bezüglich des Farbtons sichtbar. Während mit dem erfindungsgemäßen Haarfärbemittel 1c und dem cremeförmigen Haartönungsmittel 1a etwa gleiche Farbtöne erzielt werden, erhält man mit dem herkömmlichen schaumförmigen Haartönungsmittel eine deutliche Farbabweichung.

Beispiele für erfindungsgemäße Haartönungsmittel

**Beispiel 2: Schwarzes Haartönungsmittel in Schaumform**

| | | |
|---|---|---|
| 1,20 g | Cetylstearylalkohol |
| 0,40 g | Laurylsulfat-Natriumsalz |
| 0,25 g | Parfüm |
| 5,00 g | Ethanol, 96prozentig |
| 1,00 g | 4-N-Ethyl-1,4-bis-[(2'-hydroxyethyl)-amino]-2-nitrobenzol |
| 0,30 g | 4-[Bis-(2'-hydroxyethyl)-amino]-1-(2'-hydroxy ethylamino)-2-nitrobenzol |
| 0,10 g | 4-(2'-Hydroxyethylamino)-3-nitrotoluol |
| 0,05 g | 4-(2'-Ureidoethylamino)-nitrobenzol |
| 0,20 g | 1-(2'-Hydroxy-4'-sulfo-6'-nitro)-naphthylazo-2-hydroxynaphthalin |
| 0,10 g | 1-[4'-Bis-(2"-hydroxyethyl)-amino]-phenylazo]-4-aminobenzol |
| 91,40 g | Wasser |
| 100,00 g | |

Die vorstehende Wirkstoff-Lösung wird mit Ammoniak auf pH 9 eingestellt, im Verhältnis 92 : 8 mit Propan/Butan (2,7 bar) in eine Aerosoldose abgefüllt. Anschließend werden 30 g des Mittels als Schaum auf dunkle menschliche Haare aufgetragen. Nach einer Einwirkungszeit von 20 Minuten bei Raumtemperatur wird das Haar mit Wasser gespült und getrocknet. Das Haar ist intensiv schwarz gefärbt.

**Beispiel 3: Braunes Haartönungsmittel in Schaumform**

| | | |
|---|---|---|
| 1,00 g | Cetylalkohol |
| 1,60 g | Lauryldiglykolethersulfat-Natriumsalz, 28prozentige Lösung |
| 0,50 g | Parfüm |
| 0,30 g | 4-N-Ethyl-1,4-bis-[(2'-hydroxyethyl)-amino]-2-nitrobenzol |
| 0,04 g | 4-(2'-Ureidoethylamino)-nitrobenzol |
| 0,05 g | 4-(2'-Hydroxyethylamino)-3-nitrotoluol |
| 0,05 g | Tetraaminoanthrachinon |
| 0,05 g | 1-[4'-Bis-[(2"-hydroxyethyl)-amino]-phenylazo]-4-aminobenzol |
| 0,20 g | 4-[Bis- (2'-hydroxyethyl)-amino]-1-(2'-hydroxyethylamino)-2-nitrobenzol |
| 96,21 g | Wasser |
| 100,00 g | |

Die vorstehende Wirkstoff-Lösung wird mit Ammoniak auf pH 9 eingestellt, im Verhältnis 92 : 8 mit einer Mischung aus n-Butan und Dimethylether (8 : 2) in eine Aerosoldose abgefüllt und als Schaum entnommen. 20 g des Schaums werden auf blonde menschliche Haare aufgetragen. Nach einer Einwirkungszeit von 30 Minuten bei Raumtemperatur wird gespült und getrocknet. Das Haar hat eine dunkelbraune Farbe angenommen

**Beispiel 4: Mittelblondes Haartönungsmittel in Schaumform**

| | | |
|---|---|---|
| 3,00 g | Cetylstearylalkohol |
| 1,00 g | Laurylsulfat-Natriumsalz |
| 0,40 g | 4-N-Ethyl-1,4-bis-[(2'-hydroxyethyl)-amino]-2-nitrobenzol |
| 0,02 g | 4-(2'-Hydroxyethylamino)-3-nitrotoluol |
| 0,01 g | 4-(2',3'-Dihydroxypropylamino)-3-nitro-trifluormethylbenzol |
| 0,02 g | 4-(2'-Ureidoethylamino)-nitrobenzol |
| 0,10 g | 1-[4'-[Bis(2''-hydroxyethyl)-amino]-phenylazo]-4-aminobenzol |
| 95,45 g | Wasser |
| 100,00 | |

Die vorstehende Wirkstoff-Lösung wird mit Ammoniak auf pH 9 eingestellt, im Verhältnis 90 : 10 mit Dimethylether in eine Aerosoldose abgefüllt und als Schaum entnommen. 50 g des Schaumsw werden auf blonde menschliche Haare aufgetragen und nach einer Einwirkungszeit von 20 Minuten bei Raumtemperatur wird das Haar mit Wasser gespült. Das Haar hat eine natürliche mittelblonde Färbung erhalten.

**Beispiel 5: Haselnußblondes Haartönungsmittel in Schaumform**

```
1,10 g    Cetylstearylalkohol
0,40 g    Laurylsulfat-Natriumsalz
0,30 g    4-N-Ethyl-1,4-bis-[(2'-hydroxyethyl)-amino]-2-
          nitrobenzol
0,15 g    4-(2'-Hydroxyethylamino)-3-nitrotoluol
0,05 g    4-(2'-Ureidoethylamino)-nitrobenzol
0,20 g    4-(2',3'-Dihydroxypropylamino)-5-chlor-2-
          nitroanilin
97,80 g   Wasser
100,00 g
```

Der pH-Wert der Wirkstoff-Lösung wird auf 6,5 eingestellt. Anschließend wird die Lösung im Verhältnis 94 : 6 mit Propan/Butan (2,7 bar) in eine Aerosoldose abgefüllt. 40 g des Schaums werden aus dieser Aerosoldose auf blonde menschliche Haare aufgetragen. Nach einer Einwirkungszeit von 15 Minuten bei 45 Grad Celsius unter einer Wärmehaube wird das Haar mit Wasser gespült und getrocknet. Das Haar hat eine natürliche blonde Farbe mit rötlichem Überschein angenommen.

Alle Prozentangaben dieser Anmeldung stellen, sofern nicht anders angegeben, Gewichtsprozente dar.

**Patentansprüche**

1. Mittel zum Tönen von Haaren, auf der Basis einer wäßrigen oder wäßrig-alkoholischen Farbstoffträgermasse und darin gelösten direktfärbenden Haarfarbstoffen, welches in Schaumform angewendet wird dadurch gekennzeichnet, daß es

   0,5 - 5,0 Gewichtsprozent eines $C_{14}$-$C_{18}$-Fettalkohols oder eines Gemisches aus $C_{14}$-$C_{18}$-Fettalkoholen und

   0,1 - 1,5 Gewichtsprozent eines $C_8$-$C_{16}$-Alkylsulfats oder eines mit 1 bis 4 Ethylenoxideinheiten oxethylierten $C_8$-$C_{16}$-Alkylethersulfats

   sowie

   0,1 - 1,5 Gewichtsprozent 4-N-Ethyl-1,4-bis-[(2'-hydroxyethyl)-amino]-2-nitro benzol,

   0,01 - 0,25 Gewichtsprozent 4-(2'-Ureidoethylamino)-nitrobenzol und

   0,01 - 0,5 Gewichtsprozent 4-(2'-Hydroxyethylamino)-3-nitrotoluol

   enthält, wobei die Gewichtsprozentangaben sich jeweils auf die Gesamtmenge des Mittels beziehen.

2. Mittel nach Anspruch 1, dadurch gekennzeichnet, daß es 0,1 - 15 Gewichtsprozent Ethanol oder Isopropanol enthält.

3. Mittel nach Anspruch 1 oder 2, dadurch gekennzeichnet, daß der Fettalkohol oder das Gemisch aus Fettalkoholen ausgewählt ist aus $C_{16}$-$C_{18}$-Fettalkoholen.

4. Mittel nach einem der Ansprüche 1 bis 3, dadurch gekennzeichnet, daß der Fettalkohol oder das Gemisch aus Fettalkoholen in einer Menge von 0,5 - 3,0 Gewichtsprozent enthalten ist.

5. Mittel nach einem der Ansprüche 1 bis 4, dadurch gekennzeichnet, daß das Alkylsulfat oder das Alkylethersulfat ein $C_{10}$-$C_{14}$-Alkylsulfat oder $C_{10}$-$C_{14}$-Alkylethersulfat ist.

6. Mittel nach einem der Ansprüche 1 bis 5, dadurch gekennzeichnet, daß das Alkylsulfat oder das Alkylethersulfat in einer Menge von 0,1 - 1,0 Gewichtsprozent enthalten ist.

7. Mittel nach einem der Ansprüche 1 bis 6, dadurch gekennzeichnet, daß es weitere direktfärbende Farbstoffe enthält, welche ausgewählt sind aus 4-[Bis-(2'-hydroxyethyl)-amino]-1-(2'-hydroxyethylamino)-2-nitrobenzol, 4-(2′,3′-Dihydroxypropylamino)-3-nitro-trifluormethylbenzol, 4-(2′,3′-Dihydroxypropylamino)-5-chlor-2-nitroanilin, 4-(3′-Hydroxypropylamino)-3-nitrophenol, 1-[4′-Bis-[(2″-hydroxyethyl)-amino]-phenylazo]-4-aminobenzol, 2-Amino-4,6-dinitrophenol, 1-Amino-2-(2′-hydroxyethylamino)-5-nitrobenzol, 1,4,5,8-Tetraaminoanthrachinon, 1-(2′-Hydroxy-4′-sulfo-6′-nitro)naphthylazo-2-hydroxy-naphthalin, 1,4-Diaminoanthrachinon, 1,4-Diamino-2-methoxyanthrachinon und 1-Methylamino-4-(2′-hydroxyethylamino)anthrachinon.

8.  Mittel nach einem der Ansprüche 1 bis 7, dadurch gekennzeichnet, daß es einen pH-Wert von 4 bis 10 aufweist.

9.  Mittel nach einem der Ansprüche 1 bis 8, dadurch gekennzeichnet, daß es im Verhältnis 20 : 1 bis 1 : 1 zusammen mit einem Treibgas, welches ausgewählt ist aus Propan, Butan, Dimethylether oder deren Gemischen, in einen Druckbehälter abgefüllt und in Form eines Aerosolschaums entnommen wird.

10. Verfahren zum Tönen von Haaren, dadurch gekennzeichnet, daß man eine zur Tönung des Haares ausreichende Menge des schaumförmigen Haartönungsmittels nach einem der Ansprüche 1 bis 9 auf das Haar aufträgt, sodann den Schaum in das Haar einarbeitet und nach einer Einwirkungszeit von 5 bis 30 Minuten bei 15 bis 50 Grad Celsius das Haar mit Wasser spült und trocknet.

## Claims

1.  Hair toning agent based on an aqueous or aqueous-alcoholic colorant carrier and direct-acting hair colorants dissolved therein, which is used in foam form, characterised in that it contains
    0.5 - 5.0 weight % of a $C_{14}$-$C_{18}$ fatty alcohol or a mixture of $C_{14}$-$C_{18}$ fatty alcohols; and
    0.1 - 1.5 weight % of a $C_8$-$C_{16}$ alkyl sulphate or a $C_8$-$C_{16}$ alkyl ether sulphate oxyethylated with 1 to 4 ethylene oxide units;
    as well as
    0.1 - 1.5 weight % of 4-N-ethyl-1,4-bis-[(2′-hydroxyethyl)-amino]-2-nitrobenzene;
    0.01 - 0.25 weight % of 4-(2′-ureidoethylamino)-nitrobenzene; and
    0.01 - 0.5 weight % of 4-(2′-hydroxethylamino)-3-nitrotoluene,
    the weight percent indications referring in each case to the total amount of the agent.

2.  Agent according to Claim 1, characterised in that it contains 0.1 - 15 weight % of ethanol or isopropanol.

3.  Agent according to Claim 1 or 2, characterised in that the fatty alcohol or the mixture of fatty alcohols is selected from the group comprising $C_{16}$-$C_{18}$ fatty alcohols.

4.  Agent according to any one of Claims 1 to 3, characterised in that the fatty alcohol or mixture of fatty alcohols is present in an amount of 0.5 - 3.0 weight %.

5.  Agent according to any one of Claims 1 to 4, characterised in that the alkyl sulphate or alkyl ether sulphate is a $C_{10}$-$C_{14}$ alkyl sulphate or a $C_{10}$-$C_{14}$ alkyl ether sulphate.

6.  Agent according to any one of Claims 1 to 5, characterised in that the alkyl sulphate or alkyl ether sulphate is present in an amount of 0.1 to 1.0 weight %.

7.  Agent according to any of Claims 1 to 6, characterised in that it contains further direct-acting colorants which are selected from the group comprising 4-[bis-(2′-hydroxyethyl)-amino]-1-(2′-hydroxyethyl-amino)-2-nitrobenzene, 4-(2′,3′-dihydroxypropylamino)-3-nitro-trifluoromethylbenzene, 4-(2′,3′-dihydroxypropylamino)-5-chloro-2-nitroaniline, 4-(3′-hydroxypropylamino)-3-nitrophenol, 1-[4′-bis-[(2″-hydroxyethyl)-amino]-phenylazo]-4-aminobenzene, 2-amino-4,6-dinitrophenol, 1-amino-2-(2′-hydroxyethylamino)-5-nitrobenzene, 1,4,5,8-tetraaminoanthraquinone, 1-(2′-hydroxy-4′-sulpho-6′-nitro)naphthylazo-2-hydroxy-naphthaline, 1,4-diaminoanthraquinone, 1,4-diamino-2-methoxyanthraquinone and 1-methylamino-4-(2′-hydroxyethylamino)anthraquinone.

8.  Agent according to any one of Claims 1 to 7, characterised in that it has a pH of 4 to 10.

9.  Agent according to any one of Claims 1 to 8, characterised in that it is decanted into a pressurised container in a ratio of 20 : 1 to 1 : 1 together with a propellant which is selected from propane, butane, dimethylether or mixtures thereof, and is discharged in the form of an aerosol foam.

10. Process for toning hair, characterised in that an amount of the foam-forming hair toning agent according to any one of Claims 1 to 9 sufficient for toning the hair is applied to the hair, the foam is then worked into the hair and, after a reaction time of 5 to 30 minutes at 15 to 50°C, the hair is rinsed with water and dried.

**Revendications**

1. Produit pour donner une nuance aux cheveux, à base d'une masse support de teinture, aqueuse ou aqueuse-alcoolique et de substances de coloration des cheveux, à action colorante directe, y étant dissoutes, produit utilisé sous forme de mousse, caractérisé en ce qu'il contient :

   de 0,5 à 5,0 pourcent en poids d'un alcool gras en $C_{14}$ à $C_{18}$ ou d'un mélange d'alcools gras en $C_{14}$ à $C_{18}$, et

   de 0,1 à 1,5 pourcent en poids d'un alkylsulfate en $C_8$ à $C_{16}$ ou d'un alkyléther-sulfate en $C_8$ à $C_{16}$, oxéthylé avec 1 à 4 unités d'oxyde d'éthylène,

   ainsi que

   de 0,1 à 1,5 pourcent en poids de 4-N-ethyl-1,4-bis[(2'-hydroxyéthyl)-amino]-2-nitrobenzène,

   de 0,01 à 0,25 pourcent en poids de 4-(2'-ureidoéthylamino)-nitrobenzène et

   de 0,01 à 0,5 pourcent en poids de 4-(2'-hydroxyéthylamino)-3-nitrotoluène,

   où les indications en poids se rapportent chaque fois au poids total du produit.

2. Produit selon la revendication 1, carartérisé en ce qu'il contient de 0,1 à 15 pourcent en poids d'éthanol ou d'isopropanol.

3. Produit selon la revendication 1 ou 2, caractérisé en ce que l'alcool gras ou le mélange d'alcools gras est sélectionné parmi les alcools gras en $C_{16}$ à $C_{18}$.

4. Produit selon l'une des revendications 1 à 3, caractérisé en ce que l'alcool gras ou le mélange d'alcools gras est contenu en une quantité allant de 0,5 à 3,0 pourcent en poids.

5. Produit selon l'une des revendications 1 à 4, caractérisé en ce que l'alkylsulfate ou l'alkyléthersulfate est un alkylsulfate en $C_{10}$ à $C_{14}$ ou alkyléthersulfate en $C_{10}$ à $C_{14}$.

6. Produit selon l'une des revendications 1 à 5, caractérisé en ce que l'alkylsulfate ou l'alkyléthersulfate est contenu en une quantité allant de 0,1 à 1,0 pourcent en poids.

7. Produit selon l'une des revendications 1 à 6, caractérisé en ce qu'il contient d'autres substances colorants à action colorante directe, choisies parmi le 4-[bis-(2'-hydroxyéthyl)amino]-1-(2'-hydroxyéthylamino)-2-nitrobenzène, 4-(2',3'-dihydroxypropylamino)-3-nitro-trifluorométhylbenzène, 4-(2',3'-dihydroxypropylamino)-5-chloro-2-nitroaniline, 4-(3'-hydroxypropylamino)-3-nitrophénol, 1-[4'-bis-[(2''-hydroxyéthyl)-amino-phénylazo]-4-aminobenzène, 2-amino-4,6-dinitrophénol, 1-amino-2-(2'hydroxyéthylamino)-5-nitrobenzène, 1,4,5,8-tétraaminoanthrachinone, 1-(2'-hydroxy-4'-sulfo-6'-nitro)naphtylazo-2-hydroxy-naphtaline, 1,4-diaminoanthrachinone, 1,4-diamino-2-méthoxyanthrachinone et 1-méthylamino-4-(2'-hydroxyéthylamino)antrachinone.

8. Produit selon l'une des revendications 1 à 7, caractérisé en ce qu'il présente un pH allant de 4 à 10.

9. Produit selon l'une des revendications 1 à 8, caractérisé en ce qu'il est conditionné dans un rapport allant de 20 : 1 à 1 : 1, conjointement avec un gaz vecteur, qui est sélectionné parmi le propane, le butane, le diméthyléther ou leurs mélanges, dans un réservoir à pression, et prélevé sous forme d'une mousse aérosol.

10. Procédé pour donner une nuance aux cheveux, caractérisé en ce qu'on applique sur les cheveux le produit renforcateur de nuance des cheveux selon l'une des revendications 1 à 9, sous forme de mousse, en quantité suffisante pour obtenir un renforcement de la nuance des cheveux, qu'on fait ensuite pénétrer la mousse dans les cheveux et qu'après avoir laissé agir pendant 5 à 30 minutes à une température allant de 15 à 50°C, on rince les cheveux à l'eau et on les sèche.